(19) Europäisches Patentamt
European Patent Office
Office européen des brevets

(11) Numéro de publication: **0 244 329**
**A2**

(12) # DEMANDE DE BREVET EUROPEEN

(21) Numéro de dépôt: **87420099.1**

(22) Date de dépôt: **14.04.87**

(51) Int. Cl.$^3$: **C 07 C 45/49**
C 07 C 47/52, C 07 D 317/54

(30) Priorité: **28.04.86 FR 8606364**

(43) Date de publication de la demande:
**04.11.87 Bulletin 87/45**

(84) Etats contractants désignés:
**AT BE CH DE ES GB GR IT LI LU NL SE**

(71) Demandeur: **RHONE-POULENC CHIMIE**
**25, quai Paul Doumer**
**F-92408 Courbevoie Cédex(FR)**

(72) Inventeur: **Bluthe, Norbert**
**39, rue Clément Michut**
**F-69100 Villeurbanne(FR)**

(72) Inventeur: **Perron, Robert**
**La Pecolière**
**F-69390 Charly(FR)**

(74) Mandataire: **Vignally, Noel et al,**
**RHONE-POULENC INTERSERVICES Service Brevets**
**Chimie Centre de Recherches de Saint-Fons B.P. 62**
**F-69192 Saint-Fons Cedex(FR)**

(54) **Procédé de préparation d'aldéhydes aromatiques.**

(57) Procédé d'obtention d'aldéhydes aromatiques par réaction d'un mélange monoxyde de carbone/hydrogène avec un halogénure aromatique du groupe des bromures et iodures, en présence d'un catalyseur à base d'un métal noble, d'une base azotée et éventuellement d'un agent complexant du métal noble pris dans le groupe des phosphines et des phosphites, caractérisé en ce que le rapport $CO/H_2$ est inférieur à 1.

EP 0 244 329 A2

Croydon Printing Company Ltd.

# PROCEDE DE PREPARATION D'ALDEHYDES AROMATIQUES

La présente invention a pour objet un procédé de préparation d'aldéhydes aromatiques par hydrocarbonylation d'halogénures aromatiques en présence d'un métal noble, d'une base azotée tertiaire et d'un dérivé du phosphore.

Dans le brevet américain No. 3.960.932 on a décrit un procédé de préparation d'aldéhydes consistant à faire réagir un mélange gazeux d'hydrogène et de monoxyde de carbone avec un halogénure organique du groupe des halogénures aromatiques, vinyliques et hétérocycliques en présence d'une amine tertiaire et d'un catalyseur au palladium consistant en un complexe d'un dérivé du palladium divalent avec une phosphine (triphénylphosphine), un phosphite ou une arsine ou en l'association d'un sel de palladium divalent (acétate, chlorure) ou de palladium métallique finement divisé avec un agent complexant du groupe des phosphines, des phosphites ou des arsines. Dans ce dernier cas, le rapport nombre d'at-g de P/nombre d'at.g de Pd peut être compris entre 0,5 et 5. La réaction est conduite à une température de 75 à 175°C et sous une pression de 7 à 140 bar. Ce procédé convient tout spécialement bien à la préparation d'aldéhydes aromatiques par hydrocarbonylation des bromures correspondants. En dépit des bons rendements auxquels il conduit, ce procédé souffre d'un grave inconvénient qui réside dans des durées de réaction de l'ordre de 10 à 26 heures. Ces durées de réaction se traduisent par de faibles productivités de l'appareillage qui otent tout intérêt industriel au procédé.

Afin de pallier les inconvénients du procédé décrit dans le brevet américain 3.960.932, on a proposé dans la demande de brevet européen No. 0.109.606 de conduire l'hydrocarbonylation des halogénures aromatiques à des pressions de 20 à 400 bar, à une température de 80 à 250°C, en présence d'un catalyseur à base de métal noble, d'une base tertiaire azotée et d'une quantité importante d'une phosphine ou d'un phosphite. La quantité de dérivé phosphoré représente de 2 à $10^5$ fois la quantité molaire de catalyseur, et de préférence de 10 à 1000 fois. En permettant le recours à des températures de réaction élevées sans dégradation du catalyseur, ce procédé permet d'augmenter la vitesse de la

réaction et par conséquent la productivité de l'appareillage. Toutefois l'augmentation de la vitesse de la réaction s'avère insuffisante et liée à la mise en oeuvre de températures élevées.

Dans les procédés de l'art antérieur, on a recours à des pressions aussi élevées que possible (en particulier dans la demande européenne précitée, il est indiqué que la pression totale doit être d'au moins 20 bar) et le rapport $CO/H_2$ dans le mélange gazeux n'est pas considéré comme critique ; de préférence ce rapport est égal à 1.

Dans le but d'élever la vitesse de la réaction la demanderesse s'est livrée à une étude systématique des conditions de la réaction qui lui a permis de constater, de façon surprenante, que le rapport $CO/H_2$ exerce une influence sur la vitesse de la réaction.

Plus spécifiquement, la présente invention a pour objet un procédé d'obtention d'aldéhydes aromatiques par réaction d'un mélange monoxyde de carbone/hydrogène avec un halogénure aromatique du groupe des bromures et iodures, en présence d'un catalyseur à base d'un métal noble, d'une base azotée et éventuellement d'un agent complexant du métal noble pris dans le groupe des phosphines et des phosphites, caractérisé en ce que le rapport $CO/H_2$ est inférieur à 1.

On a en effet constaté que pour une pression totale donnée, la vitesse de la réaction est d'autant plus élevée que la pression partielle d'oxyde de carbone est plus faible et que pour une pression d'oxyde de carbone déterminée, la vitesse de la réaction augmente en même temps que la pression d'hydrogène. La demanderesse a en outre constaté que le choix d'un rapport $CO/H_2$ judicieux permet à la réaction de se dérouler avec une excellente vitesse à des pressions totales relativement faibles, par exemple inférieures à 20 bar. Enfin l'influence de la diminution de la pression partielle d'oxyde de carbone sur la vitesse de la réaction est d'autant plus importante que la concentration en base dans le milieu réactionnel est plus élevée. Ceci constitue un autre aspect de la présente invention. De préférence le rapport $CO/H_2$ est inférieur ou égal à 0,9 et plus préférentiellement encore à 0,8.

La pression totale du mélange gazeux peut varier dans de larges limites et prendre les valeurs données dans l'art antérieur. Plus spécifiquement, la pression totale peut varier dans un intervalle de 1 à

250 bar et de préférence de 10 à 150 bar. Cependant la pression totale dépend dans une certaine mesure des pressions partielles de monoxyde de carbone et d'hydrogène. En effet, comme cela a été exposé précédemment, la vitesse de la réaction est d'autant plus élevée que la pression partielle de CO est plus faible. De ce point de vue, il est en général préférable de conduire la réaction à des pressions partielles de CO inférieures ou égales à 20 bar et de préférence à 15 bar. La valeur inférieure limite de la pression de CO est celle à partir de laquelle, dans les conditions de la réaction, la vitesse de passage de l'oxyde de carbone de la phase gazeuse dans la phase liquide devient inférieure à la vitesse de réaction de l'oxyde de carbone avec l'halogénure aromatique. La pression partielle d'oxyde de carbone peut être aussi faible que 0,5 bar. De préférence elle est comprise dans un intervalle de 1 à 20 bar. Bien que la vitesse de la réaction augmente avec la pression partielle d'hydrogène, il n'est pas souhaitable de dépasser des valeurs à partir desquelles des réactions secondaires telle que l'hydrogénolyse du brome ou de l'iode soient favorisées au dépend de la réaction d'hydrocarbonylation. La pression partielle d'hydrogène doit donc être choisie de façon à assurer à la fois, compte-tenu des autres conditions de réaction, une vitesse aussi élevée de la réaction et une sélectivité aussi élevée que possible. En général il n'est pas nécessaire de recourir à une pression partielle d'hydrogène supérieure à 100 bar. La limite inférieure de la pression partielle d'hydrogène dépend de la valeur choisie pour la pression partielle d'oxyde de carbone. En général, la pression partielle d'hydrogène peut être aussi faible que 2 bar, de préférence 5 bar. Dans ces conditions la pression totale de réaction dépend des pressions partielles de CO et de $H_2$ choisies.

Comme cela a été exposé auparavant, l'influence des pressions partielles de CO et de $H_2$ sur la vitesse de la réaction est d'autant plus perceptible que la concentration en base azotée tertiaire est plus élevée. Il est préférable que la concentration en base azotée exprimée en mole/litre de mélange halogénure aromatique/base et le cas échéant solvant soit maintenue pendant la durée de la réaction à une valeur au moins égale à 2 et, de préférence à au moins 2,5.

Les autres conditions de la réaction sont celles décrites dans

l'état de la technique.

Ainsi, on peut utiliser à titre de catalyseurs pour la mise en oeuvre du procédé selon l'invention un métal noble finement divisé du groupe VIII de la classification périodique des éléments tels que le palladium, le rhodium ou l'iridium, ou leurs sels d'acides minéraux ou organiques ou leurs complexes avec des composés donneurs de doublets électroniques, en particulier leurs complexes avec les phosphines, les phosphites ou les arsines. Les dérivés du palladium conviennent tout particulièrement bien à la mise en oeuvre du procédé selond l'invention. Comme exemples spécifiques de dérivés du palladium, on peut citer les carboxylates (acétate, propionate, butyrate, benzoate) de palladium (II), le chlorure palladeux et les complexes du palladium de formules générales $PdX_2[P(R)_3]_2$ ou $PdX_2[P(OR)_3]_2$ dans lesquelles X représente un atome d'halogène (brome, chlore) ou un reste d'un acide minéral ou carboxylique et R un radical hydrocarboné. On peut citer en particulier le dichlorodi(triphénylphosphino)palladium (II) et le dibromodi(tritolylphosphino)palladium (II).

La quantité de catalyseur exprimée en atome-gramme de métal ou en mole de dérivé métallique par mole d'halogénure aromatique peut varier dans de larges limites. Ainsi elle peut être comprise entre $10^{-5}$ et $10^{-1}$ at.g ou moles par mole et de préférence entre $10^{-4}$ et $10^{-2}$ at.g ou moles par mole.

A titre de base azotée tertiaire, on peut faire appel à des amines de formule générale :

$$N - (R_1)_3$$

dans laquelle les radicaux $R_1$, identiques ou différents, représentent des restes hydrocarbonés comportant de 1 à 20 atomes de carbone tels que les radicaux alkyle, cycloalkyle ou aryle. De préférence les symboles $R_1$ représentent des radicaux alkyles comportant de 1 à 10 atomes de carbone ou des radicaux cycloalkyles ayant de 5 à 10 atomes de carbone. Comme exemple de telles bases on peut citer la triéthylamine, la tri-n-propylamine, la tri-n-butylamine, la méthyldibutylamine, la méthyldicyclohexylamine, l'éthyldiisopropylamine. On peut encore faire

appel à des bases tertiaires, hétérocycliques telles que la pyridine et les picolines.

La quantité de base doit être suffisante à la fois pour neutraliser l'hydracide libéré par la réaction et pour que la concentration en base reste au moins égale à 2 moles par litre de mélange réactionnel pendant la durée de la réaction. Lorsque ces deux conditions sont satisfaites, il n'y a pas de limites supérieures critiques de la quantité de base qui peut être utilisée en large excès par rapport à la quantité théoriquement nécessaire à la neutralisation de l'hydracide formé. Pour maintenir la concentration en base au moins égale aux valeurs limites données précédemment pendant toute la durée de la réaction, la quantité de base doit être calculée pour qu'en fin de réaction la concentration en base soit au moins égale à ces valeurs. On peut également ajouter une quantité de base supplémentaire au fur et à mesure du déroulement de la réaction pour compenser celle consommée par la neutralisation de l'hydracide.

Les phosphines et les phosphites qui conviennent au déroulement de la réaction sont ceux cités dans le brevet américain 3.960.932 ou dans la demande européenne 0.109.606 dont le contenu est inclu par référence. On peut citer comme exemples non limitatifs de ces composés : la triphénylphosphine, le triphénylphosphite, la diéthylphénylphosphine, le diéthylphénylphosphite, la tritolylphosphine, le tritolylphosphite, la trinaphtylphosphine, le trinaphtylphosphite, la diphénylméthylphosphine, le diphénylméthylphosphite, la diphénylbutylphosphine, le diphénylbutylphosphite, la tris-(p-méthoxycarbonylphényl)-phosphine, le tris-(p-méthoxycarbonylphényl)-phosphite, la tris-(p-cyanophényl)-phosphine, le tris-(cyanophényl)-phosphite, le triéthylphosphite, la tributylphosphine, le tributylphosphite.

La présence d'un agent complexant phosphoré libre dans le milieu réactionnel dépend de la nature du catalyseur et/ou des conditions de la réaction. Lorsque le catalyseur est constitué par un complexe d'un métal noble avec une phosphine ou un phosphite, la présence de ces derniers à l'état libre n'est pas indispensable. Toutefois elle s'avère avantageuse lorsque la réaction est conduite à température élevée, par exemple à température supérieure à 150°C. Lorsqu'on met en oeuvre un métal noble à

l'état métallique ou un dérivé non complexé par une phosphine ou un phosphite, tel que les carboxylates de métaux nobles par exemple, il est nécessaire d'opérer en présence du composé phosphoré. Lorsqu'on conduit la réaction en présence d'un phosphite ou d'une phosphine la quantité en est choisie de façon à ce que le rapport du nombre d'atome-gramme de phosophore (P) au nombre d'atome-gramme de métal (M) soit au moins égal à 2. Le rapport P/M peut prendre des valeurs aussi élevées que 10.000. Un rapport P/M compris entre 5 et 1000 est en général convenable.

Le procédé selon l'invention convient tout spécialement bien à la préparation d'aldéhydes aromatiques de formule générale :

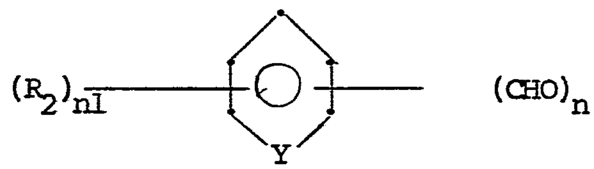

(I)

dans laquelle :

- n est 1 ou 2 ;
- $n_1$ est un nombre entier de 1 à 4 ;
- $R_2$ représente un atome d'hydrogène, de fluor ou de chlore, un radical alkyle éventuellement substitué par un ou plusieurs atomes de chlore et/ou de fluor, cycloalkyle, aryle, alkoxy, cycloalkoxy, aryloxy, alkoxycarbonyle, cycloalkoxycarbonyle, aryloxycarbonyle, alkyl-, cycloalkyl- ou arylcarbonyloxy, éventuellement substitués par un ou plusieurs atomes de fluor et/ou de chlore, nitrile ou, lorsque $n_1$ est égal à 2, deux radicaux $R_2$ portés par des atomes de carbone vicinaux forment avec ces derniers un cycle hydrocarboné ou un hétérocycle.

Lorsque $n_1$ est supérieur à 1, les différents substituants $R_2$ peuvent être identiques ou différents.

- Y représente un reste divalent -CH- ou un atome d'azote.

Dans la formule (I) $R_2$ représente plus particulièrement :

. des radicaux alkyles linéaires ou ramifiés comportant de 1 à 20 atomes de carbone tels que les radicaux méthyle, éthyle, propyles, butyles, pentyles, éthyl-2 hexyle, trifluorométhyle, difluorochlorométhyle, trichlorométhyle, décyles. De préférence $R_2$

représente un radical alkyle inférieur (ayant de 1 à 4 atomes de carbone).

. des radicaux cycloalkyles comportant de 5 à 10 atomes de carbone : cyclopentyle, cyclohexyle, cyclooctyle.

. des radicaux phényles éventuellement substitués par un ou plusieurs radicaux alkyles inférieurs ou alkoxy inférieurs, tels que phényle, xylyles, toluyles, méthoxyphényles, éthoxyphényles.

. des radicaux alkoxy comportant de 1 à 20 atomes de carbone, de préférence de 1 à 10, tels que les radicaux méthoxy, éthoxy, isopropyloxy, butoxy, trifluorométhoxy, difluorochlorométhoxy, trichlorométhoxy.

. des radicaux alkoxycarbonyles comportant de 1 à 10 atomes de carbone dans le reste alkoxy, de préférence des radicaux alkoxycarbonyles inférieurs tels que méthoxy-, éthoxy-, isopropyloxy-, butyloxycarbonyle.

. des radicaux cycloalkoxycarbonyles comportant de 5 à 10 atomes de carbone, tels que les radicaux cyclopentyloxycarbonyle, cyclohexyloxycarbonyle.

. des radicaux phényloxycarbonyle, tolyloxycarbonyle.

. des radicaux alkylcarbonyloxy comportant de 1 à 10 atomes de carbone tels que acétoxy, propionyloxy, butyroyloxy.

. des radicaux cycloalkylcarbonyloxy ayant de 5 à à 10 atomes de carbone tels que cyclopentanoyloxy, cyclohexanoyloxy.

. des radicaux benzoyloxy, méthylbenzoyloxy, diméthylbenzoyloxy.

. lorsque deux radicaux $R_2$ forment un cycle avec les atomes de carbone vicinaux qui les portent, ce cycle peut être plus particulièrement un cycle benzènique éventuellement substitués par des radicaux alkyle ou alkoxy inférieurs, ou un cycle méthylènedioxy (dioxa-1,3 cyclopentane).

Comme exemples spécifiques d'aldéhydes de formule (I) qui peuvent être préparés par le procédé de l'invention on peut citer : le benzaldéhyde, les tolualdéhydes, les anisaldéhydes, la vanilline, le triméthoxybenzaldéhyde, le méthylènedioxy-3,4 benzaldéhyde (pipéronal), le téréphtalaldéhyde, les o-, m- ou p-trifluorométhoxybenzaldéhydes, les o-, m- ou p-trichlorométhoxybenzaldéhydes, les o-, m- ou p-difluorochlorométhoxybenzaldéhydes, les naphtylaldéhydes, les formylpyridines.

Comme halogénures aromatiques constituant les produits de départ du procédé selon l'invention, on peut faire appel à des produits de formule générale :

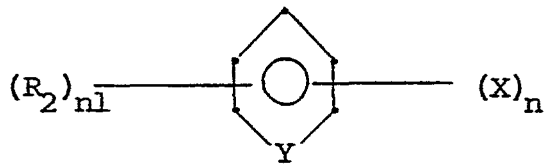

(II)

dans laquelle n, $n_1$, $R_2$ et Y ont la signification donnée ci-avant et X représente un atome de brome ou d'iode. A titre d'exemples non limitatifs de tels composés, on peut citer : le bromobenzène, les o-, m- et p-bromotoluènes, le diméthyl-2,3 bromobenzène, le diméthyl-2,4 bromobenzène, les o-, m- et p-trifluorobenzènes, les o-, m- et p-fluoroiodobenzènes, le difluoro-2,3 bromobenzène, les o-, m- et p-trifluorométhylbromobenzènes, les o-, m- et p-trifluorométhyliodobenzènes, les trifluorométhoxybromobenzènes, les trifluorométhoxyiodobenzènes, les o-, m- et p-difluorochlorométhylbenzènes, les difluorochlorométhoxybromobenzènes, les bromobenzonitriles, les dibromobenzènes, le bromo-1 naphtalène, la bromo-2 pyridine, la bromo-4 pyridine, le p-bromodiphényléther, les bromobenzoates de méthyle, la p-bromoanisole, l'orthobromoanisole, la p-bromophénétole, le diméthoxy-3,4 bromobenzène, le triméthoxy-3,4,5 bromobenzène, le bromo-3 méthylènedioxybenzène.

La température à laquelle est mis en oeuvre le procédé selon l'invention peut varier dans de larges limites. En général une température prenant toute valeur de l'intervalle de 50 à 250°C convient bien. De préférence la température est comprise entre 75 et 200°C.

Le procédé selon la présente invention est conduit en phase liquide. On peut faire appel le cas échéant à un solvant inerte dans les conditions de la réaction. A cet effet on peut utiliser des hydrocarbures aliphatiques ou cycloaliphatiques saturés (hexane, cyclohexane) ou aromatiques : benzène, toluène, xylène ; des esters tels que le benzoate de méthyle, le téréphtalate de méthyle, l'adipate de méthyle, le phtalate de dibutyle, des esters ou des éthers de polyols tels que le diacétate de

tétraéthylèneglycol, des éthers cycliques (tétrahydrofuranne, dioxane).

D'un point de vue pratique le procédé selon l'invention est mis en oeuvre de façon simple par introduction dans un autoclave inerte de l'halogénure d'aryle, de la base azotée, du catalyseur et le cas échéant du dérivé phosphoré et d'un solvant puis alimentation dans l'autoclave fermé d'une pression adéquate d'un mélange $CO/H_2$. Le contenu de l'autoclave est ensuite porté sous agitation à la température convenable jusqu'à ce que l'absorption de gaz cesse. La pression dans l'autoclave peut être maintenue constante pendant la durée de la réaction en le reliant à une réserve de mélange gazeux à la pression choisie. En fin de réaction le contenu de l'autoclave est refroidi, l'autoclave dégazé et la masse réactionnelle est filtrée pour séparer l'halohydrate de la base azotée. Le filtrat est ensuite distillé pour séparer les constituants organiques du milieu réactionnel. Le résidu de distillation contenant le catalyseur peut être recyclé pour une nouvelle opération.

Le procédé selon l'invention peut être mis en oeuvre en continu ou en discontinu.

Les exemples suivants illustrent l'invention et montrent comment elle peut être mise en pratique.

Dans ces exemples la vitesse de la réaction a été exprimée soit en nombre de millimolécules de gaz absorbé par heure ($mmol.h^{-1}$), soit en nombre de bar de gaz absorbé par heure ($bar.h^{-1}$). Comme la vitesse de la réaction est d'ordre zéro par rapport à l'halogénure aromatique, donc indépendante de la concentration en substrat dans le milieu réactionnel, l'hydrocarbonylation n'a pas été poursuivie jusqu'à son terme ; la réaction se déroulant pratiquement à la même vitesse durant toute sa durée, il a suffi de mesurer cette vitesse après un temps limité de contact des réactifs.

EXEMPLE 1

Dans un réacteur en acier inoxydable (marque commerciale HASTELLOY B2) résistant à la pression, muni d'un dispositif de chauffage et agité par un agitateur de type CAVITATOR, on charge :

- 74 g (368 mmoles) de bromométhylènedioxy-3,4 benzène
- 40 mg (0,179 mmole) de diacétate de palladium
- 3,77 (14,4 mmoles) de triphénylphosphine
- 64 ml de toluène
- 18 ml de phtalate de dibutyle.
- 80 g (792 mmoles) de triéthylamine (110 ml), soit une concentration de 3,35 moles/l

Le réacteur est ensuite fermé et purgé à l'aide d'un mélange gazeux comportant 1 volume de CO pour 3 volumes d'hydrogène. On charge 4 bar de pression de ce mélange et porte le contenu du réacteur à 170°C puis on ajuste la pression à 30 bar à l'aide du même mélange.

La pression dans le réacteur est maintenue constante par alimentation du réacteur avec un mélange équivolumique de $CO/H_2$. La chute de pression dans la réserve est suivie. Après 2 h 30 de réaction on arrête l'alimentation en mélange gazeux et refroidit le contenu du réacteur. L'analyse par chromatographie en phase gazeuse du ciel gazeux dans le réacteur (phase gazeuse surmontant la phase liquide) montre que le rapport $CO/H_2$ est égal à 1/3.

Compte-tenu de la chute de pression enregistrée dans la réserve pendant la durée de l'essai la vitesse initiale de la réaction est de 64 $bar.h^{-1}$.

ESSAI COMPARATIF

On a répété l'expérience précédente avec un rapport $CO/H_2$ de 1, la pression totale étant de 30 bar.

La vitesse initiale de la réaction exprimée en $bar.h^{-1}$ s'est élevée à 44.

La comparaison de cet essai avec l'exemple qui le précède montre que toutes choses étant égales par ailleurs, la vitesse de la réaction augmente quand le rapport $CO/H_2$ passe de 1 à 1/3.

EXEMPLE 2

On a répété l'exemple 1 avec un rapport $CO/H_2$ de 3/17.

Dans ces conditions la vitesse initiale de la réaction s'est

élevée à 88 bar.$h^{-1}$.

EXEMPLE 3

Dans un autoclave en acier inoxydable (marque commerciale HASTELLOY B2) de 125 ml on charge :

- 20,1 g (100 mmol) de bromométhylènedioxy-3,4 benzène
- 11,2 mg (0,05 mmol) de diacétate de palladium
- 1,05 g (4 mmol) de triphénylphosphine
- 20 ml d'un mélange toluène/phtalate de dibutyle dans un rapport volumique de 3/1.
- 22,26 g (220 mmol) de triéthylamine (31 ml), soit une concentration de 3,49 moles/l.

On introduit ensuite 2 bar d'oxyde de carbone puis 7 bar d'hydrogène. Le contenu du réacteur agité par un système de va et vient est porté à 170°C puis relié à une réserve contenant un mélange oxyde de carbone/hydrogène 1/1 dont la chute de pression permet de suivre le déroulement de la réaction. La pression dans l'autoclave est maintenue à 15 bar pendant la durée de l'expérience. On maintient ces conditions pendant 4 heures. Le contenu de l'autoclave est dégazé. La vitesse initiale d'absorption du mélange $CO/H_2$ s'est élevée à 47 mmoles.$h^{-1}$. Il s'est formé 7,56 g de piperonal et le taux de convertion du bromométhylènedioxybenzène s'est élevé à 56 %.

EXEMPLE 4

On a procédé selon l'exemple 3 mais avec une pression partielle de 1 bar de CO et une pression partielle de $H_2$ de 8 bar. On a interrompu l'expérience après 2 heures dans ces conditions.

La vitesse initiale d'absorption du mélange $CO/H_2$ s'est élevée à 67 m.mol.$h^{-1}$. Il s'est formé 5,94 g de pipéronal pour un taux de transformation du bromométhylènedioxybenzène de 44 %.

REVENDICATIONS

1°) Procédé d'obtention d'aldéhydes aromatiques par réaction d'un mélange monoxyde de carbone/hydrogène avec un halogénure aromatique du groupe des bromures et iodures, en présence d'un catalyseur à base d'un métal noble, d'une base azotée et éventuellement d'un agent complexant du métal noble pris dans le groupe des phosphines et des phosphites, caractérisé en ce que le rapport $CO/H_2$ est inférieur à 1.

2°) Procédé selon la revendication 1, caractérisé en ce que le rapport $CO/H_2$ est inférieur ou gél à 0,9.

3°) Procédé selon l'une quelconque des revendications 1 à 2, caractérisé en ce que la pression totale est comprise entre 1 et 250 bar.

4°) Procédé selon l'une quelconque des revendications 1 à 3, caractérisé en ce que la pression d'oxyde de carbone est inférieure ou égale à 20 bar.

5°) Procédé selon l'une quelconque des revendications 1 à 4, caractérisé en ce que la concentration en base azotée, exprimée en mole par litre de mélange réactionnel est maintenue à une valeur d'au moins 2 moles/litre pendant la durée de la réaction.

6°) Procédé selon l'une quelconque des revendications 1 à 5, caractérisé en ce que l'on prépare des aldéhydes aromatiques de formule générale :

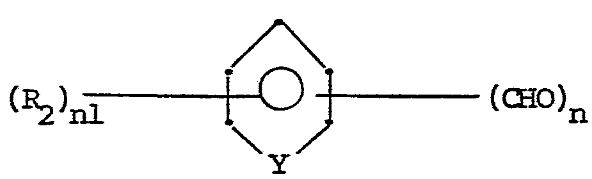 (I)

dans laquelle :

- n est 1 ou 2 ;

- $n_1$ est un nombre entier de 1 à 4 ;

- $R_2$ représente un atome d'hydrogène, de fluor ou de chlore, un radical alkyle éventuellement substitué par un ou plusieurs atomes de chlore et/ou de fluor, cycloalkyle, aryle, alkoxy, cycloalkoxy, aryloxy, alkoxycarbonyle, cycloalkoxycarbonyle, aryloxycarbonyle, alkyl-, cycloalkyl- ou arylcarbonyloxy, éventuellement substitués par un ou plusieurs atomes de fluor et/ou de chlore, nitrile ou, lorsque $n_1$ est égal à 2, deux radicaux $R_2$ portés par des atomes de carbone vicinaux forment avec ces derniers un cycle hydrocarboné ou un hétérocycle ;

- Y représente un reste divalent -CH- ou un atome d'azote ;

à partir d'halogénures d'aryle de formule générale :

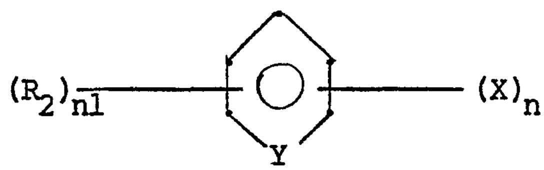

(II)

dans laquelle n, $n_1$, $R_2$ et Y ont la signification donnée ci-avant et X représente un atome de brome ou d'iode.

7°) Procédé selon l'une quelconque des revendications 1 à 6, caractérisé en ce que la base azotée tertiaire est une amine.

8°) Procédé selon l'une quelconque des revendications 1 à 7, caractérisé en ce que la température est comprise dans un intervalle de 50 à 250°C.

9°) Procédé selon l'une quelconque des revendications 1 à 8, caractérisé en ce que le catalyseur est constitué par du palladium métallique ou un dérivé du palladium.

10°) Procédé selon l'une quelconque des revendications 1 à 9, caractérisé en ce que la quantité de palladium exprimée en atome-gramme de métal noble ou en mole de dérivé métallique par mole d'halogénure aromatique est comprise dans un intervalle de $10^{-5}$ à $10^{-1}$.

11°) Procédé selon l'une quelconque des revendications 1 à 10, caractérisé en ce que la quantité de dérivé phosphoré est telle que le rapport du nombre des atome-gramme de phosphore au nombre des atome-gramme de métal soit compris dans un intervalle de 1 à 10.000.